# EUROPEAN PATENT SPECIFICATION

(11) **EP 1 392 320 B1**
(45) Date of publication and mention of the grant of the patent: **10.01.2007**
(21) Application number: 02769291.2
(22) Date of filing: 26.04.2002
(51) Int. Cl.: A61K 31/568, A61K 31/5685, A61K 31/57, A61K 31/565, A61P 5/02

(54) **17-METHYLENE-ANDROSTAN-3a-OL ANALOGS AS CRH INHIBITORS**
17-METHYLEN-ANDROSTAN-3a-OL-ANALOGA ALS CRH- INHIBITOREN
ANALOGUES DE17-METHYLENE-ANDROSTAN-3a-OL COMME INHIBITEURS DE LA CRH

(30) Priority: 03.05.2001 US 848247
(43) Date of publication of application: 03.03.2004
(73) Proprietor: Pherin Pharmaceuticals, Inc., Redwood City, California 94063 (US)
(72) Inventor: MONTI, Louis, Salt Lake City, UT 84108 (US); BERLINER, David, L., Atherton, CA 94027 (US); JENNINGS-WHITE, Clive, L., Salt Lake City, UT 84019 (US); ADAMS, Nathan, W., West Jordan, UT 84088 (US)
(74) Representative: Barz, Peter
(86) International application number: PCT/US2002/013255
(87) International publication number: WO 2002/089814

(56) References cited:
- EP-A- 0 850 902
- US-A- 5 969 168
- US-A- 5 994 333

## Description

### BACKGROUND OF THE INVENTION

### FIELD OF THE INVENTION

This invention relates to the use of certain 17-methyleneandrostan-3α-ol analogs as corticotropin releasing hormone (CRH) inhibitors.

### DESCRIPTION OF RELATED ART

US Patent No. 5,969,168 discloses a number of androstane derivatives (including 17-methyleneandrostanols such as 17-methyleneandrost-4-en-3α-ol) as vomeropherins, i.e. compounds that act on the vomeronasal organ of a human to alter hypothalamic or autonomic function.

US Patents Nos. 5,563,131 and 6,057,439 disclose a number of pregnane derivatives (including pregna-17-en-3-ols such as pregna-17(20)Z-en-3α-ol and pregna-4,17(20)Z-dien-3α-ol) as vomeropherins.

Corticotropin releasing hormone (CRH), also known as corticotropin releasing factor (CRF) is a hypothalamic releasing hormone that stimulates the production of adrenocorticotrophic hormone (ACTH) from the anterior pituitary and thereby modulates the hypothalamic-pituitary-adrenal axis (HPA) function. In extra-hypothalamic brain areas, CRH is believed to be the major physiologic regulator of the autonomic, immune, and behavioral effects of stress. CRH is believed to be hypersecreted in patients with major depression: evidence supporting this suggestion includes (1) cerebrospinal fluid CRH is increased in drug-free depressed patients; (2) post-mortem analysis of brain tissue indicates an increased number of cells expressing CRH in the hypothalamus of depressed patients; (3) the number of CRH receptor sites in the frontal cortex is reduced in depressed suicide victims compared with controls, which is likely to be a result of CRH hypersecretion; and (4) the ACTH response to CRH is blunted in depressed patients, probably due to downregulation of CRH receptors. In laboratory animals, CRH appears to have both depressogenic and anxiogenic properties. On the basis of these findings, it has been hypothesized that CRH antagonists may comprise a novel class of antidepressant and anxiolytic compounds.

Hypersecretion of CRH in the brain may contribute to the symptomatology seen in such neuropsychiatric disorders as depression, anxiety-related disorders, and anorexia nervosa. Over production of CRH may also lead to peripheral inflammation at sites such as the synovial joints, contributing to autoimmune diseases such as rheumatoid arthritis.

Recently published data in animals implicate CRH in mediating stress and the response to stress, e.g. in irritable bowel syndrome. There is a longstanding hypothesis that hypercortisolism may precipitate affective changes, and that reduction of corticosteroid concentrations or receptor activity would be a way to the treatment of depression, and some studies suggest such a possibility.

Finally, CRH and the adrenocorticosteroids have been reported to have effects on sleep. Cortisol enhances slow wave sleep (SWS) and suppresses raid eye movement REM) sleep. Regulating CRH secretions could have an important effect on the quality of sleep, including treating the profound sleep disturbances that are common in depression and anxiety-related disorders.

### SUMMARY OF THE INVENTION

In a first aspect, this invention is the use of a compound of formule I for the manufacture of a medicament for treating a disease treatable by inhibition of CRH in a human suffering therefrom comprising administration to the vomeronasal organ of the human an effective amount of a compound of formula I: 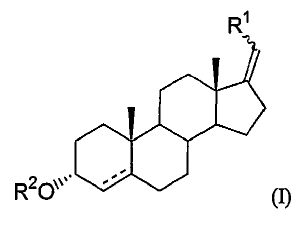 where:
R¹ is hydrogen or methyl;
R² is hydrogen, alkyl, or R'CO, where R' is alkyl or phenyl;
the dashed line indicates an optional double bond; and
the wavy line indicates the Z or E isomer.

In a second aspect, this invention is an improved synthesis of 17-methyleneandrosta-4-en-3α-ol.

### DETAILED DESCRIPTION OF THE PREFERRED EMBODIMENTS

### Definitions

"Alkyl" means a hydrocarbyl group having from one to four carbon atoms, or a branched or cyclic hydrocarbyl group having from three to four carbon atoms. Exemplary alkyl groups include methyl, ethyl, isopropyl, cyclopropyl, *tert*-butyl, and cyclopropymethyl.

"Pharmaceutically acceptable excipient " means an excipient that is useful in preparing a pharmaceutical composition that is generally safe, non-toxic, and desirable. Such excipients may be solid, liquid, semisolid, or, in the case of an aerosol composition, gaseous.

A "therapeutically effective amount" means the amount that, when administered to the vomeronasal organ of a human suffering from a disease, such as depression, is sufficient to effect treatment for that disease, but which amount is insufficient to have a systemic effect on the disease by absorption into the circulation.

"Treating" or "treatment" of a disease, such as depression, includes preventing the disease from occurring in a human that may be predisposed to the disease but does not yet experience or exhibit symptoms of the disease (prophylactic treatment), inhibiting the disease (slowing or arresting its development), providing relief from the symptoms of the disease (including palliative treatment), and relieving the disease (causing regression of the disease).

The compound of this invention may possess one or more chiral centers, and can therefore be produced as individual stereoisomers or as mixtures of stereoisomers, depending on whether individual stereoisomers or mixtures of stereoisomers of the starting materials are used. Unless indicated otherwise, the description or naming of a compound or group of compounds is intended to include both the individual stereoisomers or mixtures (racemic or otherwise) of stereoisomers. Methods for the determination of stereochemistry and the separation of stereoisomers are well known to a person of ordinary skill in the art [*see* the discussion in Chapter 4 of March: *Advanced Organic Chemistry,* 4th ed., John Wiley and Sons, New York, NY, 1992].

Implicit hydrogen atoms are omitted from the formulae for clarity, but should be understood to be present

### Presently Preferred Compounds

While the broadest definition of the invention is set out in the Summary of the Invention, certain compounds of this invention are presently preferred.

Preferred compounds are those where:
(1) if R¹ is methyl, the compound is the Z-isomer; and
(2) where R² is hydrogen.

Presently preferred compounds useful in this invention include: 17-methyleneandrosta-4-en-3α-ol, and pregna-17(20)Z-en-3α-ol.

### Pharmacology and Utility

The compounds of this invention are, when administered to the VNO, CRH inhibitors. Their activity as CRH inhibitors in vivo can be measured directly by measurement of the inhibition of CRH or indirectly by the measurement of other hormones, such as ACTH, and steroids, such as cortisol, associated with CRH, as discussed in the Examples.

The compounds of this invention, because of their activity as CRH inhibitors, find utility in the treatment of diseases in which inhibition of CRH is therapeutic. For example, while the gonadotrophic releasing hormone (GnRH) neuronal system releases GnRH in a pulsatile fashion, with a periodicity in adults of between 60 and 100 minutes, peripherally administered CRH suppresses GnRH pulse generator activity. Delivery of the compounds of this invention to the VNO will lower the levels of CRH and will therefore increase GnRH pulse generator activity, which will subsequently activate the pituitary-gonadal function. Diseases thus treatable include: delayed puberty, cryptorchidism, functional hypothalamic amenorrhea, lack of sexual drive, lack of orgasm, precocious puberty, endometriosis, and hormone-dependent tumors such as uterine leiomyoma, breast cancer, and prostate cancer.

The compounds of this invention have particular utility as antidepressants. In particular, they are expected to have the following advantages over conventional antidepressants:
(1) rapid onset of action, because of the direct local delivery of the compound to vomeronasal organ receptors and consequent action. Current antidepressant drugs (typically orally administered) are known to take from days to, typically, weeks for therapeutic effectiveness to be achieved;
(2) lack of systemic effects or toxicity, because of the very low (picogram to nanogram) doses used and the local route of administration;
(3) lack of suppression of sexual behavior, because CRH suppression will lead to GnRH enhancement and a consequent increase in pituitary gonadotropin release. Most current antidepressant drugs negatively affect sexual behavior (decrease in sexual drive, erectile function, and orgasm).

### Pharmaceutical compositions and administration

In general, compounds of this invention will be administered in therapeutically effective amounts to the vomeronasal organ, either singly or in combination with at least one other compound of this invention. A therapeutically effective amount may vary widely depending on the disease and its severity, the age and relative health of the human being treated, the potency of the compound(s), and other factors. Therapeutically effective amounts of compounds of this invention range from approximately 20 picograms (pg) to 20 nanograms (ng); for example, 200 pg to 2 ng., when administered directly to the vomeronasal organ. Correspondingly higher doses may be used when the compounds are administered by general nasal inhalation or applied to the nasal passages by ointment, cream or gel, or impregnated on a pad, for example, or when the compounds are applied to the facial skin near the nose, also for example in an ointment, cream, or gel. A person of ordinary skill in the art will be able without undue experimentation, having regard to that skill and this disclosure, to determine a therapeutically effective amount of a compound of this invention for a given disease.

In general, compounds of this invention will be administered as pharmaceutical compositions by the vomeronasal route [i.e. by intranasal administration such that at least a portion of the dose administered contacts the vomeronasal organ]. Compositions may take the form of solutions, suspensions, aerosols, or any other appropriate compositions; and comprise at least one compound of this invention in combination with at least one pharmaceutically acceptable excipient Suitable excipients are well known to persons of ordinary skill in the art, and they, and the methods of formulating the compositions, may be found in such standard references as Alfonso: *Remington's Pharmaceutical Sciences,* 17th ed., Mack Publishing Company, Easton PA, 1985. The amount of a compound of this invention in the composition may vary widely depending on the type of composition, size of a unit dosage, kind of excipients, and other factors well known to those of ordinary skill in the art. In general, the final composition may comprise from 0.000001 percent by weight (%w) to 10 %w of the compound of this invention, preferably 0.00001 %w to 1 %w, with the remainder being the excipient or excipients.

The compounds or compositions of this invention may also be administered with at least one other agent useful for treatment of the disease being treated, e.g. another antidepressant, or an agent which potentiates or otherwise modulates the effect of the compounds of this invention in treating that disease. Such other agent may be another vomeropherin, which may be co-administered with the compounds of this invention, or may be an agent administered by more usual methods, such as orally or parenterally.

### Preparation of the Compounds of this Invention

The starting materials and reagents used in preparing these compounds are either available from commercial suppliers such as Aldrich Chemical Company (Milwaukee, WI) and Steraloids, Inc. (Newport, RI), or are prepared by methods well known to a person of ordinary skill in the art following procedures described in such references as Fieser and Fieser's *Reagents for Organic Synthesis,* vols. 1-17, John Wiley and Sons, New York, NY, 1991; Rodd's *Chemistry of Carbon Compounds,* vols. 1-5 and supps, Elsevier Science Publishers, 1989; *Organic Reactions,* vols. 1-40, John Wiley and Sons, New York, NY, 1991; March: *Advanced Organic Chemistry,* 4th ed., John Wiley and Sons, New York, NY, 1992; and Larock: *Comprehensive Organic Transformations,* VCH Publishers, 1989. These schemes are merely illustrative of some methods by which the compounds of this invention can be synthesized, and various modifications to these schemes can be made and will be suggested to a person of ordinary skill in the art having regard to this disclosure.

The starting materials, intermediates, and compounds of this invention may be isolated and purified using conventional techniques, including filtration, distillation, crystallization and chromatography. They may be characterized using conventional methods, including physical constants and spectral data.

Unless specified to the contrary, the reactions described herein take place at atmospheric pressure over a temperature range between about 0 °C and 125 °C.

The compounds of this invention may be prepared by the methods described below and as given in the Examples.

Two general reaction schemes are shown below; Reaction Scheme 1 producing compounds in which R¹ is H, and showing the inversion of the 3-hydroxy group; and Reaction Scheme 2 producing compounds in which R¹ is methyl with no inversion of the 3-hydroxy group. The use of combinations of the two reaction schemes with the two commonly available starting materials allows for the preparation of the compounds of this invention.

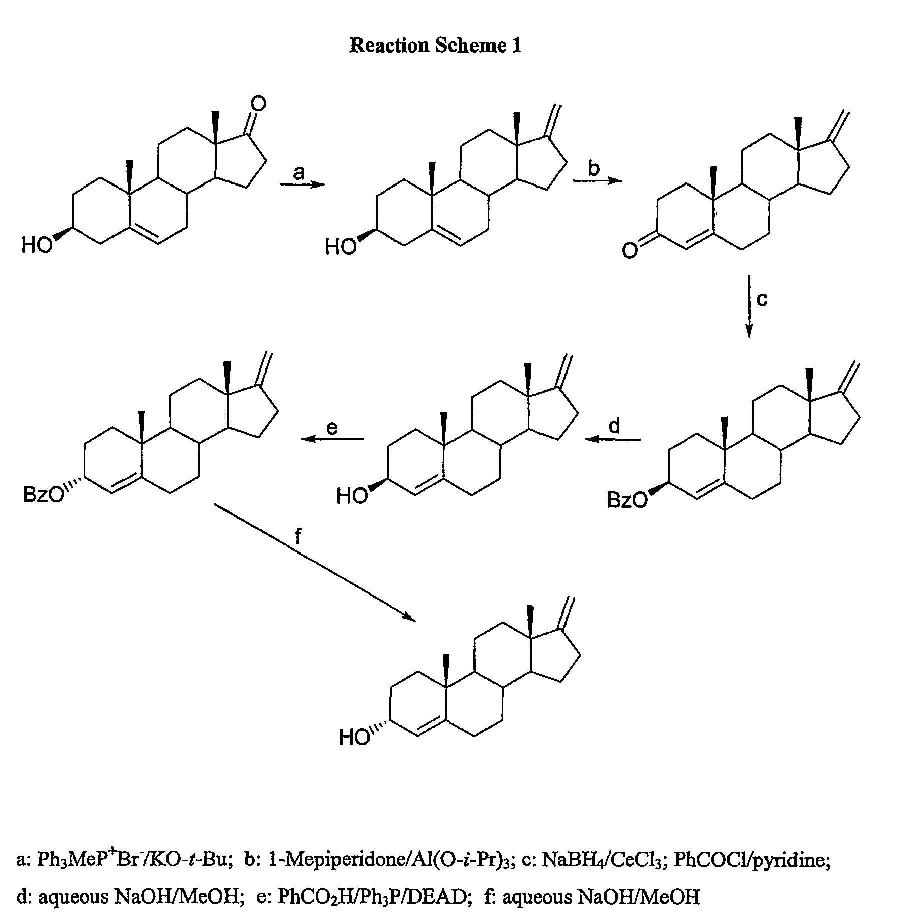

In Reaction Scheme 1, dehydroepiandrosterone is treated under Wittig reaction conditions to give 17-methyleneandrost-5-en-3β-ol; which is then oxidized and isomerized to 17-methyleneandrost-5-en-3-one; reduced (the use of a cerium(III) salt with sodium borohydride is especially beneficial in this step); optionally converted to an ester for purification and then re-hydrolyzed to the 3β-hydroxy compound (as shown in the reaction scheme), then treated under Mitsunobu reaction conditions to epimerize the 3β-hydroxy compound to an ester of 17-methyleneandrost-4-en-3α-ol, which is then hydrolyzed to give 17-methyleneandrost-4-en-3α-ol itself.

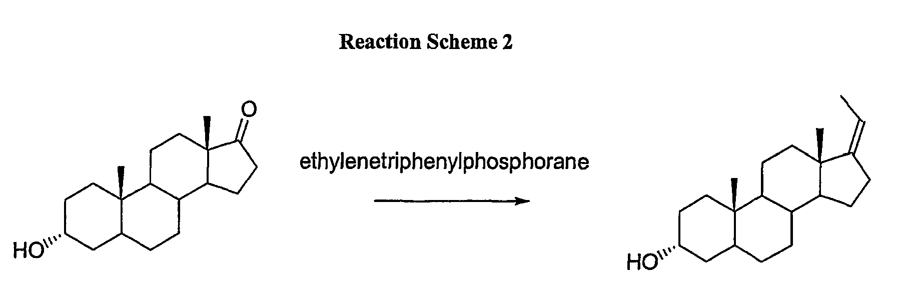

In Reaction Scheme 2, androsterone is treated under Wittig reaction conditions to give pregna-17(20)Z-en-3α-ol, with pregna-17(20)E-en-3α-ol available as a side product.

Ethers and esters of the 3-hydroxy compounds may readily be prepared by standard techniques well-known to a person of ordinary skill in the art.

A person of ordinary skill in the art, having regard to that skill, this disclosure, and the references cited herein, will be able to prepare desired compounds of this invention without undue experimentation.

### EXAMPLES

The following non-limiting examples illustrate the invention. All commercially available materials were used as received.

### Example 1. 17-Methylene-androst-4-en-3α-ol

### Step 1. 17-Methylene-5-androst-5-en-3β-ol:

Anhydrous dimethylsulfoxide (175 mL) was added to methyltriphenylphosphonium bromide (54.76 g, 0.1533 moles) and potassium *tert*-butoxide (17.20 g. 0.1533 moles) under argon and the mixture was placed in a 79-85°C bath for 1 h, giving an orange suspension. Dehydroepiandrosterone (8.84 g, 30.6 mmol) in 80 mL of anhydrous dimethylsulfoxide was then added. After stirring a further 90 min., the mixture was poured into 440 mL of ice-brine and extracted three times with 220 mL portions of heptane. The combined extracts were washed with 220 mL of acetonitrile and then filtered through Celite^{®} 503 filter aid. The residue was washed with 25 mL of heptane and the combined filtrates were concentrated under reduced pressure. Crystallization of the resulting solid from methanol gave a white powder (4.68 g, 16.3 mmol), m.p. 134-135°C (lit. m.p. 134-135°C - Macdonald et al., *Steroids,* **18***,* 753-766 (1971).), homogeneous to TLC (25% ethyl acetate/hexanes on silica gel; product R_{f} 0.31; authentic sample R_{f} 0.32).

### Step 2. 17-Methylene-androst-4-en-3-one:

17-Methyleneandrost-5-en-3β-ol (6.27 g, 21.9 mmol) was suspended in toluene (450 mL) and 1-methyl-4-piperidone (32 mL, 0.26 moles). After distilling off 75 mL of the solvent, aluminum iso-propoxide (5.36 g, 26.2 mmol) was added and the mixture was refluxed 5 hours. The cooled mixture was then washed with 300 mL of 0.5 M citric acid and 100 mL of water and filtered through Celite^{®} 503 filter aid. The residue was washed with 25 mL of toluene and the combined filtrates were concentrated under reduced pressure to give a yellow solid (6.18 g, 21.7 mmol, 99%), which TLC (25% ethyl acetate/hexanes on silica gel; R_{f} 0.45) showed was contaminated with traces of starting material (R_{f} 0.30) and a more polar contaminant (R_{f} 0.01).

### Step 3. 17-Methylene-androst-4-en-3β-yl benzoate:

To a solution of 17-methyleneandrost-4-en-3-one (3.06 g, 10.8 mmol) and cerium(III) chloride heptahydrate (40.8 mg, 0.110 mmol) in 270 mL of methanol was added sodium borohydride (0.61 g, 16 mmol). After stirring 20 min., the reaction was quenched with 15 mL of 1 N HCl and methanol was removed under reduced pressure. Saturated aqueous sodium bicarbonate (50 mL) was added and the resulting suspension was extracted three times with 50 mL portions of methylene chloride. The combined extracts were washed with 50 mL of brine, dried over magnesium sulfate, and filtered through Celite^{®} 503 filter aid. The residue was washed with 10 mL of methylene chloride and the combined filtrates were concentrated under reduced pressure. The residual white solid was taken up in 17 mL (0.21 mol) of anhydrous pyridine, benzoyl chloride (6.2 mL, 53 mmol) was added, and the mixture was stirred 16 h. The mixture was poured into 200 mL of 1 N HCl and then extracted three times into 50 mL portions of methylene chloride. The combined extracts were washed with 50 mL of 1 N HCl, 50 mL of saturated sodium bicarbonate, and 50 mL of brine, dried over magnesium sulfate, and filtered through Celite^{®} 503 filter aid. The residue was washed with 25 mL of methylene chloride and the combined filtrates were concentrated under reduced pressure. Recrystallization of the residue twice from absolute ethanol, the first time with charcoal treatment, yielded fine, white needles (2.59 g, 6.63 mmol, 61%), m.p. 154-155°C, homogeneous to TLC (5% ethyl acetate/hexanes on silica gel; product R_{f} 0.52; 1,3,5(10),16-estratetraen-3-yl methyl ether R_{f} 0.57).

### Step 4. 17-Methylene-androst-4-en-3β-ol:

Aqueous sodium hydroxide (15% w/w, 16 mL, 60 mmol) was added to 17-methylene-androst-4-en-3β-yl benzoate (2.47 g, 6.32 mmol) suspended in 320 mL of methanol. After refluxing 1 h, water (160 mL) was added and the mixture was refrigerated overnight. The resulting suspension was filtered, and the residue was washed three times with 35 mL aliquots of water and then dried overnight to give very fine white platelets (1.70 g, 5.93 mmol, 94%), m.p. 120-121°C, homogeneous to TLC (20% ethyl acetate/hexanes on silica gel; product R_{f} 0.31; 17-methyleneandrost-5-en-3β-ol R_{f} 0.27).

### Step 5. 17-Methylene-androst-4-en-3α-yl benzoate:

Diethyl azodicarboxylate (1.1 mL, 7.0 mmol) was added to 17-methyleneandrost-4-en-3β-ol (1.00 g, 3.49 mmol), triphenylphosphine (0.85 g, 7.0 mmol), and benzoic acid (1.83 g, 6.98 mmol) in 7 mL of anhydrous tetrahydrofuran over a period of 2 min. After stirring 1 h, solvent was removed under reduced pressure and the residue was taken up in 150 mL of methyl *tert*-butyl ether. The mixture was washed with 50 mL of saturated sodium bicarbonate and 50 mL of brine, dried over magnesium sulfate, and filtered through Celite^{®} 503 filter aid. The residue was washed with 10 mL of methyl *tert*-butyl ether and the combined filtrates were concentrated under reduced pressure. Flash chromatography (1 % ethyl acetate/hexanes on silica gel) of the residual solid, followed by two-fold recrystallization from absolute ethanol, the first time with charcoal treatment, yielded fine, white needles (311.2 mg, 0.797 mmol, 23%), m.p. 117-118°C, homogeneous to TLC (5% ethyl acetate/hexanes on silica gel; product R_{f} 0.50; 17-methyleneandrost-4-en-3α-yl benzoate R_{f} 0.53).

### Step 6. 17-Methylene-4-androsten-3α-ol:

Aqueous sodium hydroxide (15%, w/w, 2.0 mL, 7.5 mmol) was added to 17-methyleneandrost-4-en-3α-yl benzoate (295.0 mg, 0.7553 mmol) suspended in 40 mL of methanol. After refluxing 2 h, water (10 mL) was added to the mixture, methanol was removed under reduced pressure, and the residue was extracted three times into 10 mL portions of methyl *tert*-butyl ether. The combined extracts were washed with 10 mL of brine, dried over magnesium sulfate, and filtered through Celite^{®} 503 filter aid. The residue was washed with 5 mL of methyl *tert*-butyl ether and the combined filtrates were concentrated under reduced pressure to yield a white, crystalline solid (210.5 mg, 0.7348 mmol, 97%), m.p. 104-105°C, homogeneous to TLC (20% ethyl acetate/hexanes on silica gel; product R_{f} 0.36; 17-methyleneandrost-4-en-3α-ol R_{f} 0.30).

### Example 2. Study of 17-methyleneandrost-4-en-3α-ol

17-Methyleneandrost-4-en-3α-ol, 400 pg, or placebo were administered to the vomeronasal organs of normal adult male and female human volunteers using a delivery device which delivered the compound to the opening of the vomeronasal organ in measured quantity (two 2 second "puffs" of a 10⁻⁸ M solution of the active ingredient in propylene glycol, or propylene glycol alone). The study evaluations included objective and subjective evaluations of sleep, a power spectral analysis of the subjects' electroencephalograms, and a pencil-and-paper self-report inventory (DABS-70) of the subjects' effective state.

Results from these studies suggest that 17-methyleneandrost-4-en-3β-ol has antidepressant properties, which are not part of the present invention.

The table below provides a comparison of the results from the study with 17-methyleneandrost-4-en-3α-ol with literature reports on the effects of the effects of the marketed antidepressant paroxetine (Paxil^{®}); where changes in sleep variables are indicated as an increase (↑), decrease (↓), or no change (=) with respect to placebo.

Comparative effects of 17-methylene-4-androsten-3α-ol, 400 pg administered to the vomeronasal organ, and paroxetine, 30 mg orally, given before bedtime in normal subjects: combined effects in males and females

| **Variable Measured** | **17-Methyleneandrost-4-en-3α-ol** | **Paroxetine^{*}** |
|---|---|---|
| Sleep efficiency | ↓ | ↓ |
| Total sleep time | ↓ | ↓ |
| Sleep latency | ↑ | ↑ |
| REM sleep latency | ↑ | ↑ |
| Total REM sleep time | ↓ | ↓ |
| Stage 1 sleep time | ↑ | ↑ |
| Stage 2 sleep time | ↑ | = |
| Stages 3+4 sleep time | = | = |

| | | |
|---|---|---|
| *From Saletu et al., "Sleep Laboratory studies on the single-dose effects of the serotonin reuptake inhibitors paroxetine and flouxetine on human sleep and awakening qualities", *Sleep,* **14**(5), 439-447 (1991) | | |

The table shows great similarity in sleep profiles of the subjects treated with 17-methyleneandrost-4-en-3α-ol and with paroxetine. The increase in REM latency and the reduction of total REM sleep time are typical indicators of potential benefit in depression; in fact, many patients diagnosed with major depressive disorder may show dramatic decreases in REM sleep latency and increases in the amount of REM sleep, compared to age- and sex-matched normals.

Based on this study, 17-methyleneandrost-4-en-3α-ol shares antidepressant and stimulant properties with paroxetine and other selective serotonin reuptake inhibitor antidepressants. The antidepressant activity of 17-methyleneandrost-4-en-3α-ol is also supported by our EEG measurements, which show increased β- and θ-wave activities: similar changes are produced by the tricyclic antidepressants amitriptyline and imipramine. 17-Methyleneandrost-4-en-3α-ol also causes an increase in δ-frequency activity in stages S3 and S4 non-REM sleep, which suggests that the it may have greater effects in males than in females.

17-Methyleneandrost-4-en-3α-ol also reduces plasma concentrations of ACTH and cortisol, and also reduces cortisol concentration in urine. It also increases, relative to placebo, concentrations of the neurotransmitters norepinephrine and serotonin in blood and their metabolites in urine. These results further support the suggestion that delivery of 17-methyleneandrost-4-en-3α-ol to the vomeronasal organ produces effects similar to those of standard antidepressants.

Pregna-17(20)Z-en-3α-ol and the other compounds of the invention show similar results.

## Claims

1. The use of a compound of formula I: 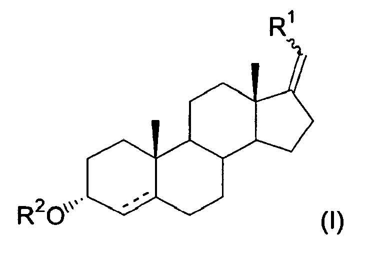 where:
R¹ is hydrogen or methyl;
R² is hydrogen, alkyl, or R'CO, where R' is alkyl or phenyl;
the dashed line indicates an optional double bond; and
the wavy line indicates the Z or E isomer,
in the manufacture of a medicament adapted for administration to the vomeronasal organ of an individual for treating delayed puberty, cryptorchidism, functional hypothalamic amenorrhea, lack of sexual drive, lack of orgasm, precocious puberty, endometriosis, and hormone-dependent tumors such as uterine leiomyoma, breast cancer, and prostate cancer in a human.

2. The use of Claim 1, where the compound is 17-methyleneandrost-4-en-3α-ol.

3. The use of Claim 1, where the compound is pregna-17(20)Z-en-3α-ol.

4. The use of Claim 1, where the effective amount is between 20 pg and 20 ng.

5. The use of Claim 4, where the effective amount is between 200 pg and 2 ng.

6. The use of Claim 1 where in formula I:
R¹ is methyl; and the wavy line indicates the Z isomer.

7. A method of preparing 17-methyleneandrost-4-en-3α-ol, comprising
(a) treating 17-methyleneandrost-4-en-3β-ol under Mitsunobu reaction conditions to give an ester of 17-methyleneandrost-4-en-3α-ol; and
(b) hydrolyzing the ester to give 17-methyleneandrost-4-en-3α-ol.

8. The method of Claim 7 where the 17-methyleneandrost-4-en-3β-ol is prepared by:
(a) treating dehydroepiandrosterone under Wittig conditions to give 17-methyleneandrost-5-en-3β-ol:
(b) oxidizing and isomerizing the 17-methyleneandrost-5-en-3β-ol to give 17-methyleneandrost-4-en-3-one; and
(c) reducing the 17-methyleneandrost-4-en-3-one to give 17-methyleneandrost-4-en-3β-ol.

9. The method of Claim 8 where the 17-methyleneandrost-4-en-3-one is reduced with sodium borohydride in the presence of a cerium(III) salt.

## Patentansprüche

1. Verwendung einer Verbindung der Formel I: 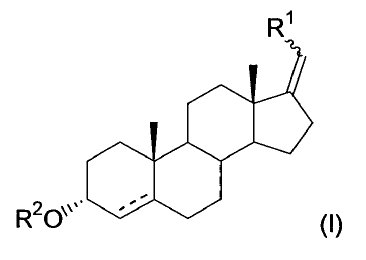 worin:
R¹ Wasserstoff oder Methyl ist;
R² Wasserstoff, Alkyl oder R'CO ist, worin R' Alkyl oder Phenyl ist;
die gestrichelte Linie eine optionale Doppelbindung angibt; und
die Wellenlinie das Z- oder E-Isomer angibt,
bei der Herstellung eines Medikaments, das geeignet ist zur Verabreichung an das Vomeronasal-Organ eines Individuums zur Behandlung von verzögerter Pubertät, Kryptorchismus, funktioneller hypothalamischer Amenorrhoe, fehlendem Sexualtrieb, Ausbleiben des Orgasmus, vorzeitiger Pubertät, Endometriose und hormonabhängigen Tumoren, wie Uterusleiomyoma, Brustkrebs und Prostatakrebs, bei einem Menschen.

2. Verwendung nach Anspruch 1, bei der die Verbindung 17-Methylenandrost-4-en-3α-ol ist.

3. Verwendung nach Anspruch 1, bei der die Verbindung Pregna-17(20)Z-en-3α-ol ist.

4. Verwendung nach Anspruch 1, bei der die wirksame Menge zwischen 20 pg und 20 ng liegt.

5. Verwendung nach Anspruch 4, bei der die wirksame Menge zwischen 200 pg und 2 ng liegt.

6. Verwendung nach Anspruch 1, bei der in Formel I:
R¹ Methyl ist; und
die Wellenlinie das Z-Isomer angibt.

7. Verfahren zur Herstellung von 17-Methylenandrost-4-en-3α-ol, umfassend
(a) Behandeln von 17-Methylenandrost-4-en-3β-ol unter Mitsunobu-Reaktionsbedingungen, um einen Ester von 17-Methylenandrost-4-en-3α-ol zu ergeben; und
(b) Hydrolysieren des Esters, um 17-Methylenandrost-4-en-3α-ol zu ergeben.

8. Verfahren nach Anspruch 7, bei dem das 17-Methylenandrost-4-en-3β-ol hergestellt wird durch:
(a) Behandeln von Dehydroepiandrosteron unter Wittig-Bedingungen, um 17-Methylenandrost-5-en-3β-ol zu ergeben;
(b) Oxidieren und Isomerisieren von 17-Methylenandrost-5-en-3β-ol, um 17-Methylenandrost-4-en-3-on zu ergeben; und
(c) Reduzieren von 17-Methylenandrost-4-en-3-on, um 17-Methylenandrost-4-en-3β-ol zu ergeben.

9. Verfahren nach Anspruch 8, bei dem das 17-Methylenandrost-4-en-3-on mit Natriumborhydrid in Anwesenheit von einem Cer(III)-Salz reduziert wird.

## Revendications

1. Emploi d'un composé de formule I : 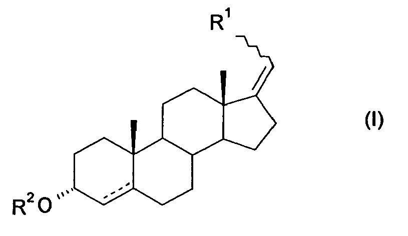 dans laquelle
- R¹ représente un atome d'hydrogène ou un groupe méthyle,
- R² représente un atome d'hydrogène, un groupe alkyle ou un groupe de formule R'CO où R' représente un groupe alkyle ou phényle,
- le trait en pointillé indique une éventuelle double liaison,
- et le trait ondulé indique l'isomère Z ou l'isomère E,
dans la fabrication d'un médicament adapté pour être administré au niveau de l'organe voméro-nasal d'un individu en vue de traiter, chez un humain, un retard de puberté, une cryptorchidie, une aménorrhée psychogène fonctionnelle, un manque de libido, une anorgasmie, une puberté précoce, une endométriose, ou une tumeur hormono-dépendante comme un fibromyome de l'utérus, un cancer du sein ou un cancer de la prostate.

2. Emploi conforme à la revendication 1, pour lequel le composé est du 17-méthylène-androst-4-én-3α-ol.

3. Emploi conforme à la revendication 1, pour lequel le composé est du prégna-17(20)Z-én-3α-ol.

4. Emploi conforme à la revendication 1, pour lequel la quantité efficace de composé vaut de 20 pg à 20 ng.

5. Emploi conforme à la revendication 4, pour lequel la quantité efficace de composé vaut de 200 pg à 2 ng.

6. Emploi conforme à la revendication 1, pour lequel, dans la formule I, R¹ représente un groupe méthyle, et l'isomère indiqué par le trait ondulé est l'isomère Z.

7. Procédé dé préparation du 17-méthylène-androst-4-én-3α-ol, qui comporte:
a) le fait de traiter du 17-méthylène-androst-4-én-3β-ol dans les conditions de la réaction de Mitsunobu, de manière à obtenir un ester de 17-méthylène-androst-4-én-3α-ol,
b) et le fait d'hydrolyser cet ester, de manière à obtenir du 17-méthylène-androst-4-én-3α-ol.

8. Procédé conforme à la revendication 7, pour lequel on prépare du 17-méthylène-androst-4-én-3β-ol :
a) en traitant de la déhydroépiandrostérone dans les conditions de la réaction de Wittig, de manière à obtenir du 17-méthylène-androst-5-én-3β-ol,
b) en opérant l'oxydation et l'isomérisation de ce 17-méthylène-androst-5-én-3β-ol, de manière à obtenir de la 17-méthylène-androst-4-én-3-one,
c) et en réduisant cette 17-méthylène-androst-4-én-3-one, de manière à obtenir du 17-méthylène-androst-4-én-3β-ol.

9. Procédé conforme à la revendication 8, dans lequel on réduit la 17-méthylène-androst-4-én-3-one au moyen de borohydrure de sodium, en présence d'un sel de cérium-III.
